# EUROPÄISCHE PATENTANMELDUNG

(11) **EP 4 640 251 A1**
(43) Veröffentlichungstag der Anmeldung: **29.10.2025**
(21) Anmeldenummer: 24171752.9
(22) Anmeldetag: 22.04.2024
(51) Int. Cl.: A61M 5/14, A61G 7/05

(54) **VORRICHTUNG ZUR ANORDNUNG AN EINEM PROFIL ODER DERGLEICHEN MIT EINEM BEFESTIGUNGSELEMENT**

(71) Anmelder: Tobii Dynavox AB, 115 50 Stockholm (SE)
(72) Erfinder: Rabasse, Kenneth, 34414 Warburg (DE); Sprunck, Annemarie, 34266 Niestetal (DE); Mackerodt, Tim, 34121 Kassel (DE)
(74) Vertreter: Hannke Bittner & Partner mbB Kassel

(57) **Zusammenfassung**

Die Erfindung betrifft eine Vorrichtung (20) zur Anordnung an einem Profil oder dergleichen mit einem Befestigungselement (1) und zeichnet sich dadurch aus, dass es wenigstens eine Aufnahme (2) für ein Zubehörteil (3) aufweist, in welche das Zubehörteil (3) aufnehmbar ist.

## Beschreibung

Die Erfindung betrifft eine Vorrichtung zur Anordnung an einem Profil oder dergleichen mit einem Befestigungselement nach dem Oberbegriff des Patentanspruchs 1. Derartige Vorrichtungen werden unter anderem dazu verwendet, um an einem Infusionsständer Infusionsbeutel einzuhängen, die Infusionslösungen enthalten, die einem Patienten verabreicht werden sollen. Derartige Vorrichtungen sind in der Regel aber fest mit dem Infusionsständer verbunden. Ein grundsätzliches Problem hierbei ist, dass Infusionsständer ein separates Bauteil sind, welches im Bereich eines technischen Pflegehilfsmittels, wie beispielsweise eines Pflegebettes, Rollstuhls oder Patientenbeistellwagen, -tisch oder -schrank, positioniert werden und unabhängig von diesem bewegt werden müssen. Da es sich hierbei um ein separat bewegbares Bauteil handelt, welches einen Platzbedarf aufweist, werden derartige Infusionsständer oftmals zu Stolperfallen, bei denen sich das Pflegepersonal Verletzungen zufügen kann, welche zudem zu Arbeitsausfällen führen können, sodass Ersatz für das Pflegepersonal bereitgestellt werden muss.

Es ist daher Aufgabe der Erfindung, eine erfindungsgemäße Vorrichtung derart weiterzuentwickeln, dass Unfälle im Pflegebereich minimiert werden und somit der Einsatz von Pflegepersonal optimiert wird.

Gelöst wird diese Aufgabe durch eine Vorrichtung mit allen Merkmalen des Patentanspruchs 1. Vorteilhafte Ausgestaltungen der Erfindung finden sich in den Unteransprüchen.

Die erfindungsgemäße Vorrichtung zur Anordnung an einem Profil oder dergleichen mit einem Befestigungselement weist dabei erfindungsgemäß eine Aufnahme für ein Zubehörteil auf, in welche das Zubehörteil aufnehmbar ist. Durch die Erfindung ist es ermöglicht, die Vorrichtung an einem Profil oder dergleichen, welches mit dem technischen Pflegehilfsmittel verbunden oder verbindbar ist anzuordnen, sodass die Vorrichtung direkt damit verbunden ist und keinen Platzbedarf auf dem Fußboden im Bereich des technischen Pflegehilfsmittels benötigt. Durch die erfindungsgemäße Ausgestaltung der Vorrichtung wird somit im Bereich des technischen Pflegehilfsmittels im Bereich des Fußbodens ein selbständig bewegbares Mobilteil vermieden, welches als Stolperfalle dienen kann und somit Ursache für Unfälle und somit Verletzungen des Pflegepersonals sein könnte. Hierdurch werden Unfälle und Ausfallzeiten des Pflegepersonals vermieden, sodass eine effizientere Pflegeleistung erfolgen kann und die Ausfallzeiten des Pflegepersonals minimiert werden. Zudem können durch die Verwendung verschiedener Zubehörteile eine Vielzahl von Anwendungen bedient werden. Exemplarisch seien hier nur Schlauchführungen, Zugentlastungen, Haken und Karabiner für die Anbringung von Infusionsbeutel oder -behälter genannt.

Die erfindungsgemäße Vorrichtung kann somit, im Gegensatz zu z.B. herkömmlichen Infusionsständern oder Schlauchhaltern und auch im Gegensatz zu an Pflegebetten befestigten Infusionsständern durch die verschiedenen Zubehörteile an jeweilige Pflegesituation angepasst werden.

Durch die erfindungsgemäße Vorrichtung wird zudem eine Reduzierung von technischen Pflegehilfsmitteln ermöglicht, die Platz auf dem Boden einnehmen. Somit kann auch die Hygiene im Pflegeumfeld deutlich verbessert werden, da die Raumpflege vereinfacht wird.

Nach einer ersten vorteilhaften Ausgestaltung der Erfindung ist es vorgesehen, dass wenigstens eine Aufnahme als Einsteckschacht ausgebildet ist, in welche das Zubehörteil einsteckbar ist. Durch diese Ausgestaltung ist es ermöglicht, entsprechende Zubehörteile in einfacher Weise einfach in die erfindungsgemäße Vorrichtung einzustecken, ohne dass weitere Arbeitsschritte notwendig sind, um entsprechende Zubehörteile mit der erfindungsgemäßen Vorrichtung zu verbinden.

In einer weiteren vorteilhaften Ausgestaltung der Erfindung ist es vorgesehen, dass die wenigstens eine Aufnahme als Einsteckschacht ausgebildet ist, in welchem das Zubehörteil unverlierbar einsteckbar ist. Hierdurch ist ermöglicht, dass das Zubehörteil sich nicht ungewollt aus dem Einsteckschacht lösen kann und somit von der erfindungsgemäßen Vorrichtung wieder getrennt wird.

In vorteilhafter Weise weist das Zubehörteil dazu zwei zueinander gewandte Flächenelemente auf, von denen ein Flächenelement in dem Einsteckschacht einführbar ist, während das andere Flächenelement an einer Außenwand des Einsteckschachtes zum Liegen kommt, wobei die Außenwand zwischen den beiden Flächenelementen eingeklemmt ist. Durch einen derartigen Klemmmechanismus kann in einfacher Weise die Verbindung zwischen Zubehörteil und erfindungsgemäßer Vorrichtung realisiert werden, ohne dass komplexe Verbindungsschritte notwendig sind. Durch einfaches Einschieben des einen Flächenelementes in den Einsteckschacht und das Anliegen des anderen Flächenelementes an der Außenwand des Einsteckschachtes wird ein einfacher Klemmmechanismus realisiert, der die Verbindung zwischen Zubehörteil und erfindungsgemäßer Vorrichtung verwirklicht.

Um die Unverlierbarkeit des Zubehörteils in verbundenem Zustand mit der Vorrichtung zu gewährleisten ist es weiterhin vorgesehen, dass an einem der Flächenelemente Rastelemente angeordnet sind, die in Gegenrastelemente der Außenwand eingreifen. Durch derartige Rast- und Gegenrastelemente wird in einfacher Weise die Unverlierbarkeit des Zubehörteils im verbundenen Zustand mit der Vorrichtung sichergestellt.

Nach einem weiteren Gedanken der Erfindung ist es vorgesehen, dass als Zubehörteile Haken, Kabelführungen, Karabiner, Zugentlastung oder dergleichen verwendet werden. Derartige Zubehörteile werden in vielfältiger Weise im Pflegebereich benötigt. So dienen die einfachen Haken zum Einhängen von Infusionsbeuteln oder dergleichen. Auch können Karabiner zum Einhängen von Infusionsbeuteln dienen, die dabei nur durch aktives Betätigen des Karabiners wieder von dem Zubehörteil bzw. der erfindungsgemäßen Vorrichtung gelöst werden können. Da im Pflegebereich oftmals eine Vielzahl von Pflegeeinrichtungen zum Einsatz kommen, die elektrische Energie benötigen, wie beispielsweise Beatmungsgeräte, Luftbefeuchter, Kommunikationsgeräte und dergleichen mehr, werden Zubehörteile auch als Kabelführungen benötigt, um ein organisiertes Führen der Kabel dieser Einrichtungen zu gewährleisten. Auch dieses organisierte Führen der elektrischen Zuleitungen dieser Einrichtungen dient einer Unfallvermeidung für das Pflegepersonal. Weiterhin können Zubehörteile auch als Zugentlastungen dienen. Dies ist insbesondere für zu pflegende Patienten sinnvoll, die künstlich beatmet werden und eine entsprechende Zuleitung für die Beatmung haben. Damit diese Zuleitung keiner mechanischen Belastung unterliegt, sind Zugentlastungen vorgesehen,-die eine mechanische Belastung des Beatmungsschlauches zwischen der Zugentlastung und dem Patienten unterbinden.

Weitere Ziele, Vorteile, Merkmale und Anwendungsmöglichkeiten der vorliegenden Erfindung ergeben sich aus der nachfolgenden Beschreibung der Ausführungsbeispiele anhand der Zeichnungen. Dabei bilden alle beschriebenen und/oder bildlich dargestellten Merkmale für sich oder in beliebiger sinnvoller Kombination den Gegenstand der vorliegenden Erfindung, auch unabhängig von ihrer Zusammenfassung in den Ansprüchen oder deren Rückbeziehung.

Es zeigen:
- Figur 1:: ein Ausführungsbeispiel einer erfindungsgemäßen Vorrichtung,
- Figur 2:: das Ausführungsbeispiel der Figur 1 in einer Explosionsdarstellung,
- Figur 3:: eine mögliche Anordnung der erfindungsgemäßen Vorrichtung an einem Pflegebett und
- Figur 4:: eine weitere mögliche Anordnung der erfindungsgemäßen Vorrichtung an einem Pflegebett

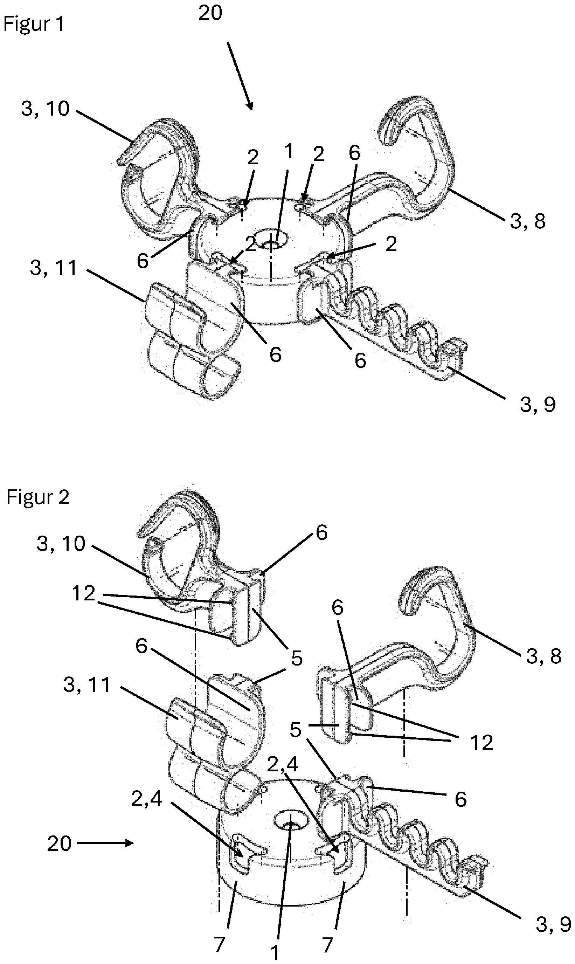

In der Figur 1 ist ein Ausführungsbeispiel einer erfindungsgemäßen Vorrichtung in einer perspektivischen Darstellung gezeigt. Dabei sind vier Zubehörteile 3 mit der erfindungsgemäßen Vorrichtung über jeweils eine Aufnahme 2, welche als Einsteckschacht 4 ausgebildet ist, verbunden. Die erfindungsgemäße Vorrichtung selbst kann dabei über ein Befestigungselement 1, welches in diesem Ausführungsbeispiel eine zentrale Öffnung für ein Profil darstellt, mit einem derartigen Profil verbunden werden. Die hier dargestellten Zubehörteile 3 sind zum einen als normaler Haken 8, als Kabelführung 9, als Karabiner 10 und als Zugentlastung 11 ausgebildet. Die Zubehörteile 3 weisen dabei zueinander gewandte Flächenelemente 5 und 6 auf, von denen jeweils das Flächenelement 5 in den Einsteckschacht 4 der jeweiligen Aufnahme 2 eingeführt ist, während das andere Flächenelement 6 an einer Außenwand 7 des Einsteckschachtes 4 zum Liegen kommt, wobei die Außenwand 7 zwischen den beiden Flächenelementen 5 und 6 eingeklemmt ist. Durch diese Klemmverbindung ist ein einfaches Verbinden der Zubehörteile mit der erfindungsgemäßen Vorrichtung ermöglicht, wobei die Zubehörteile 3 dann auch in einfacher Weise wieder von der erfindungsgemäßen Vorrichtung entnommen werden können.

In der Figur 2 ist die erfindungsgemäße Vorrichtung der Figur 1 mit den dortigen Zubehörteilen und einer perspektivischen Explosionsdarstellung gezeigt. In dieser Darstellung sind die als Einsteckschächte 4 ausgebildeten Aufnahmen 2 der erfindungsgemäßen Vorrichtung und die Zubehörteile 3 besser zu erkennen. Insbesondere ist dort auch die Außenwand 7 erkennbar, die zwischen den Flächenelementen 5 und 6 der Zubehörteile eingeklemmt wird.

In der Darstellung der Figur 2 sind nunmehr auch Rastelemente 12 zu erkennen, welche an den Flächenelementen 5 der Zubehörteile 3 angeordnet sind. Diese Rastelemente 12 greifen in hier nicht erkennbare Gegenrastelemente der erfindungsgemäßen Vorrichtung ein, sodass durch das Eingreifen der Rastelemente 12 und diese Gegenrastelemente eine Unverlierbarkeit der Zubehörteile nach dem Verbinden mit der erfindungsgemäßen Vorrichtung erreicht wird.

In dem in den Figuren dargestellten Ausführungsbeispiel ist die erfindungsgemäße Vorrichtung als kreisförmiges Zylinderelement dargestellt, welche an ihrem Außenumfang 4 Aufnahmen 2 der Zubehörteile 3 aufweist. Für andere Ausführungsbeispiele ist es auch denkbar, andere geometrische Formen und eine unterschiedliche Anzahl von Aufnahmen 2 für Zubehörteile 3 vorzusehen.

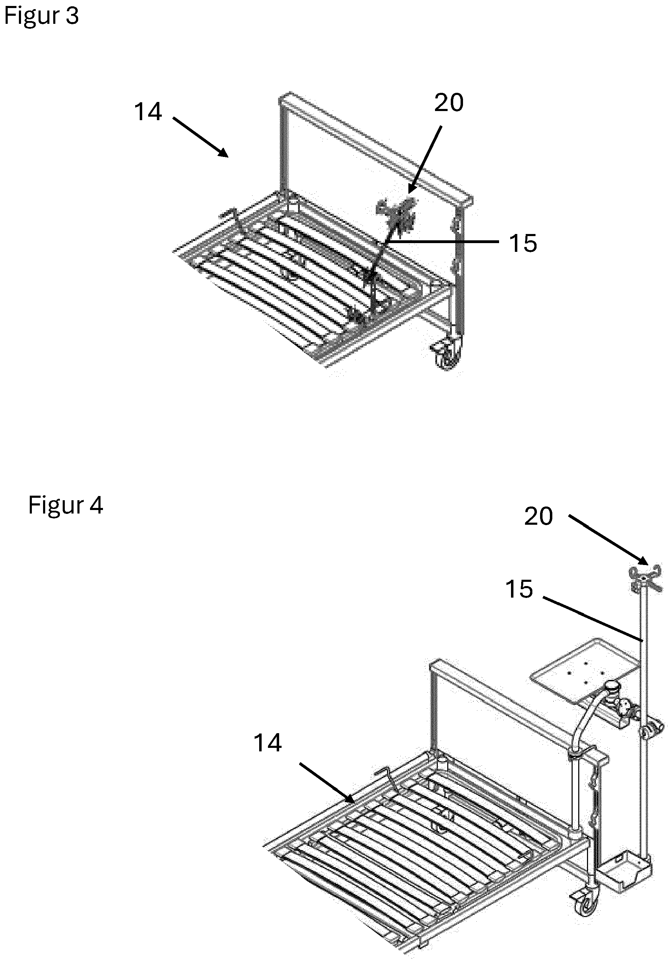

In der Figur 4 ist nunmehr dargestellt, wie die erfindungsgemäße Vorrichtung der Figuren 1 und 2 an einem als Pflegebett 14 ausgebildeten technischen Pflegehilfsmittel befestigt ist. Hierbei wird besonders verdeutlicht, dass die erfindungsgemäße Vorrichtung direkt mit dem Pflegebett 14 verbunden ist und keinen eigenständigen Ständer aufweist, der separat gehandhabt werden muss. Die erfindungsgemäße Vorrichtung ist dabei über ein Profil 15 fest mit dem Pflegebett 14 verbunden, sodass die erfindungsgemäße Vorrichtung beim Bewegen des Pflegebettes direkt mitbewegt wird und nicht separat bewegt werden muss.

In der Figur 5 ist eine andere Anordnung einer erfindungsgemäßen Vorrichtung an ein Pflegebett 14 gezeigt. Auch hierbei ist die erfindungsgemäße Vorrichtung direkt über ein Profil und mehreren Profilverbindungen mit dem Pflegebett 14 verbunden, sodass auch bei diesem Ausführungsbeispiel beim Bewegen des Pflegebettes die erfindungsgemäße Vorrichtung direkt mitbewegt wird, ohne dass ein separates Bewegen notwendig wird.

### Bezugszeichenliste

- 1: Befestigungselement
- 2: Aufnahme
- 3: Zubehörteil
- 4: Einsteckschacht
- 5: Flächenelement
- 6: Flächenelement
- 7: Außenwand
- 8: Haken
- 9: Kabelführung
- 10: Karabiner
- 11: Zugentlastung
- 12: Rastelemente
- 14: Pflegebett
- 15: Profil
- 20: Vorrichtung

## Patentansprüche

1. Vorrichtung (20) zur Anordnung an einem Profil oder dergleichen mit einem Befestigungselement (1), **dadurch gekennzeichnet, dass** es wenigstens eine Aufnahme (2) für ein Zubehörteil (3) aufweist, in welche das Zubehörteil (3) aufnehmbar ist.

2. Vorrichtung (20) nach Anspruch 1, **dadurch gekennzeichnet, dass** die wenigstens eine Aufnahme (2) als Einsteckschacht (4) ausgebildet ist, in welchen das Zubehörteil (3) einsteckbar ist.

3. Vorrichtung (20) nach Anspruch 1 oder 2, **dadurch gekennzeichnet, dass** die wenigstens eine Aufnahme (2) als Einsteckschacht (4) ausgebildet ist, in welchen das Zubehörteil (3) unverlierbar einsteckbar ist.

4. Vorrichtung (20) nach Anspruch 2 oder 3, **dadurch gekennzeichnet, dass** das Zubehörteil (3) zwei zueinander gewandte Flächenelemente (5, 6) aufweist, von denen ein Flächenelement (5) in den Einsteckschacht (4) einführbar ist, während das anderes Flächenelement (6) an einer Außenwand (7) des Einsteckschachtes (4) zum Liegen kommt, wobei die Außenwand (7) zwischen den beiden Flächenelementen (5, 6) eingeklemmt ist.

5. Vorrichtung (20) nach Anspruch 4, **dadurch gekennzeichnet, dass** an einem der Flächenelemente (5, 6) Rastelemente (12) vorgesehen sind, die in Gegenrastelemente (13) der Außenwand (7) eingreifen.

6. Vorrichtung (20) nach einem der vorhergehenden Ansprüche, **dadurch gekennzeichnet, dass** das Zubehörteil (3) als Haken (8), Kabelführung (9), Karabiner (10), Zugentlastung (11) oder dergleichen ausgebildet ist.
